# EUROPEAN PATENT APPLICATION

(11) **EP 4 821 546 A1**
(43) Date of publication of application: **14.10.2026**
(21) Application number: 25824790.7
(22) Date of filing: 15.05.2025
(51) Int. Cl.: A61N 5/10

(54) **RADIOTHERAPY DEVICE AND CONTROL METHOD THEREFOR**

(30) Priority: 25.06.2024 CN 202410823506
(71) Applicant: Sparticle Healthcare Company Limited., Beijing 100176 (CN)
(72) Inventor: LIU, Renqing, Beijing 100176 (CN); YAN, Nan, Beijing 100176 (CN); ZHUANG, Guoliang, Beijing 100176 (CN); LI, Junfeng, Beijing 100176 (CN)
(74) Representative: Loo, Chi Ching
(86) International application number: PCT/CN2025/095115
(87) International publication number: WO 2026/001403

(57) **Abstract**

Disclosed in the present application are a radiotherapy device and a control method therefor. The device comprises a fixed base, a rotating frame, a treatment beam assembly, and a fan beam CT assembly. The rotating frame is rotatably arranged on the fixed base. The treatment beam assembly and the fan beam CT assembly are arranged on the rotating frame. A rotation plane where the axis of a treatment beam of an accelerator treatment head is located is coplanar with a rotation plane where the axis of a diagnostic beam of the fan beam CT assembly is located, and the axis of the treatment beam and the axis of the diagnostic beam intersect at the rotation axis of the rotating frame. By means of the rotating frame, the treatment beam assembly and the fan beam CT assembly can be driven at the same time. Both radiotherapy and CT imaging can be realized, and during CT imaging and radiotherapy switching, a patient does not need to be displaced, thereby improving the accuracy of imaging and treatment. When a treatment working state is switched to a diagnostic working state, the treatment beam assembly is controlled to return to a preset position, so that the device that rotates at a high speed is in a dynamic balance state, thereby protecting the overall device.

## Description

### CROSS-REFERENCE TO RELATED APPLICATION

The present disclosure claims the priority of Chinese patent application filed on June 25, 2024 before the CNIPA, China National Intellectual Property Administration with the application number of 202410823506.6, and the title of "RADIOTHERAPY DEVICE AND CONTROL METHOD THEREFOR", which is incorporated herein in its entirety by reference.

### FIELD

The present disclosure relates to a radiotherapy device, particularly to a radiotherapy device with a fan beam computed tomography (CT) assembly. The present disclosure also relates to a control method for the above radiotherapy device.

### BACKGROUND

Image guidance plays an extremely important role in patient positioning and adaptive radiotherapy in radiotherapy, which makes a radiotherapy positioning process visible and accurate, and develops towards a clearer and more precise direction. Various manufacturers have successively launched products based on image guidance, such as ultrasound, cone beam computed tomography (CBCT), magnetic resonance imaging, and even positron emission tomography (PET) guided products, to achieve superior anatomical structure imaging, soft tissue imaging, or biological function imaging, so as to improve treatment accuracy. These technologies have enabled the acquisition of patient volumetric images before and during treatment, imaging and positioning of tumors, normal tissues, or patient body surface contours, achieving precise three-dimensional positioning of anatomical structures during the positioning process, and adjusting the position or plan according to position changes.

Furthermore, in order to further ensure the accuracy of image-guided positions and avoid image errors caused by patient movement, there is also a technical solution in the existing technologies that integrates an image guidance device with a radiotherapy device. However, due to the limitation of the technical principles, the existing image guidance technologies mentioned above are inadequate for meeting requirements of adaptive radiotherapy for soft tissue resolution, computed tomography (CT) value linearity, uniformity, accuracy, and other indicators, thus becoming one of bottlenecks for automatic contouring and dose calculation in the adaptive radiotherapy process.

The traditional fan beam CT, which can also achieve image guidance, is a gold standard imaging technology in radiotherapy and has high cost-effectiveness while meeting the above requirements. However, the fan beam CT requires continuous high-speed rotation for imaging, and it occupies a large space and has a heavy weight. Moreover, a rotational speed of typical radiotherapy equipment generally does not exceed 4 revolutions per minute (RPM) during radiotherapy, while CT imaging generally requires a speed between 30 RPM and 80 RPM. Therefore, when integrating the two into a single system, it is necessary to consider the stability and safety of the radiotherapy equipment at high speed. As a result, integration with the radiotherapy device remains challenging and represents a technical gap in the current existing technologies.

### SUMMARY

In view of the above problems, the present disclosure discloses a radiotherapy device and a control method therefor, which integrates a fan beam computed tomography (CT) into a radiotherapy device, realizes radiotherapy and CT imaging simultaneously through a rotation frame, and can provide high-precision image guidance without large-scale displacement of patients.

The present disclosure also discloses a control method for the above radiotherapy device.

In order to achieve the above purpose, the present disclosure adopts the following technical solutions.

The present disclosure provides a radiotherapy device, including a fixed base, a rotation frame, a treatment beam assembly, and a fan beam computed tomography (CT) assembly. The rotation frame is rotatably arranged on the fixed base. The treatment beam assembly and the fan beam computed tomography are arranged on the rotation frame. A rotation plane of an axis of a treatment beam of the accelerator treatment head is coplanar with a rotation plane of an axis of a diagnostic beam of the fan beam CT assembly, and the axis of the treatment beam and the axis of the diagnostic beam intersect at a rotation axis of the rotation frame. The treatment beam assembly includes an accelerator treatment head, a pulse modulator, a magnetron, and a treatment beam imaging unit arranged opposite to the accelerator treatment head. The fan beam CT assembly includes a fan beam CT tube and a diagnostic beam detection unit arranged opposite to the fan beam CT tube. The axis of the treatment beam is perpendicular to and coplanar with the axis of the diagnostic beam. The pulse modulator is arranged between the accelerator treatment head and the diagnostic beam detection unit, and a pulse unit of the pulse modulator is arranged along a radial direction pointing to the rotation axis. The magnetron is arranged between the accelerator treatment head and the fan beam CT tube, and is arranged along a radial direction pointing to the rotation axis.

In an exemplary embodiment of the radiotherapy device, the radiotherapy device further includes a water cooling device, a water distributor, an inlet pipe and a return pipe. The water cooling device is arranged on the rotation frame and arranged outside the treatment beam imaging unit along a radial direction pointing to the rotation axis. The water distributor is arranged on the rotation frame and between the accelerator treatment head and the fan beam CT tube, providing cooling water for heat-generating components of an accelerator. The inlet pipe and the return pipe connected between the water cooling device and the water distributor, where the inlet pipe and the return pipe are arc-shaped pipes centered on the rotation axis.

In an exemplary embodiment of the radiotherapy device, the water cooling device includes water cooling tubes and an expansion tank, and water cooling tubes is arranged along a rotational tangential direction of the rotation frame. The expansion tank is arranged parallel to the rotation axis and connected to the water cooling tubes.

In an exemplary embodiment of the radiotherapy device, the radiotherapy device further includes a shielding body movably arranged on a radial inner side of the diagnostic beam detection unit, and the shielding body has a shielding position capable of shielding the diagnostic beam detection unit when the accelerator treatment head is in operation, and an open position capable of exposing the diagnostic beam detection unit when the fan beam CT tube is in operation; a thickness of the shielding body decreases in a stepped manner from one end close to the treatment beam imaging unit.

In an exemplary embodiment of the radiotherapy device, the accelerator treatment head includes an acceleration tube, the rotation frame is provided with a fixed plate, and the fixed plate is provided with a through hole for the acceleration tube to pass through; the fixed plate is provided with a fixed ring protruding along an outer edge of the through hole, and an adjusting ring embedded inside the fixed ring and concentric with the fixed ring; the acceleration tube passes through the fixed ring and the adjusting ring, and is connected to the adjusting ring through an installation arm; the fixed ring is provided with two pairs of set screws, where the two pairs of set screws respectively pass through the fixed ring and adjustably abut against an outer side wall of the adjusting ring, to adjust a position of the acceleration tube in a horizontal direction; the installation arm at least includes three cantilevers, a connecting ring formed jointly by ends of the three cantilevers is sleeved around an exterior of the acceleration tube, and the other ends of the three cantilevers are all arranged on the adjusting ring; each of the three cantilevers is adjustably connected to the adjusting ring through a bolt assembly, to adjust a position of the acceleration tube in a vertical direction; the bolt assembly at least includes a vertical adjusting bolt and a fixed bolt that passes through an interior of the vertical adjusting bolt and is threadedly connected to the adjusting ring; two ends of the vertical adjusting bolt are respectively provided with washers, and a spring is sleeved on an exterior of the vertical adjusting bolt.

In an exemplary embodiment of the radiotherapy device, the radiotherapy device further includes:
a first driving device configured to drive the rotation frame to rotate;
where the first driving device includes a rotor and a stator;
the rotor is annular and drives the rotation frame to rotate; the rotor is provided with a plurality of magnets; the plurality of magnets are uniformly arranged along a circumferential direction of the rotor;
the stator is annular, sleeved on outside the rotor, and fixedly connected to the fixed base; the stator is provided with a plurality of sets of coils, and the plurality of sets of coils are uniformly arranged along a circumferential direction of the stator.

The present disclosure also provides a control method for a radiotherapy device, which includes: controlling the radiotherapy device to enter the treatment working state, and in the treatment working state, controlling the rotation frame to drive the treatment beam assembly to move to a treatment position for radiotherapy; controlling the radiotherapy device to enter the diagnostic working state, and in the diagnostic working state, controlling the rotation frame to drive the fan beam CT assembly to continuously rotate for diagnostic imaging. The control method further includes: before switching from the treatment working state to the diagnostic working state, controlling the treatment beam assembly to return to a preset position; and after switching to the diagnostic working state, detecting whether the treatment beam assembly is out of the preset position, and in response to detecting that the treatment beam assembly is out of the preset position, controlling the rotation frame to slow down and enter a low-speed protection state.

In an exemplary embodiment of the control method for a radiotherapy device, the step of controlling the rotation frame to enter the low-speed protection state further includes: detecting allowable force values of components arranged on the rotation frame in the rotational tangential direction; obtaining an allowable acceleration value of the rotation frame according to a minimum of the allowable force values; obtaining a maximum deceleration curve of the rotation frame according to the allowable acceleration value; and controlling the rotation frame to reduce its rotational speed according to the maximum deceleration curve and enter the low-speed protection state.

In an exemplary embodiment of the control method for a radiotherapy device, the control method further includes: before entering the treatment working state or the diagnostic working state, performing online monitoring to determine that the axis of the treatment beam and the axis of the diagnostic beam intersect at the rotation axis of the rotation frame.

In an exemplary embodiment of the control method for a radiotherapy device, the online monitoring includes:
installing a phantom to be tested at a fixed position on a treatment bed and moving a center of the phantom to be tested to a pre-calibrated isocenter position; controlling the treatment bed to perform multiple shifts along different directions, and performing imaging through the treatment beam assembly after each movement; controlling the treatment beam assembly to acquire an electronic portal imaging device (EPID) image of the phantom to be tested, registering the center of the phantom to be tested to an image center position in the EPID image, and obtaining a registration result of the phantom to be tested; controlling the treatment beam assembly to perform a Winston-Lutz test on the phantom to be tested, in combination with the registration result, analyzing and obtaining a consistency result between a radiation center of the treatment beam and the isocenter position, and adjusting the treatment beam assembly; and controlling the fan beam CT assembly to perform CT imaging on the phantom to be tested, analyzing and obtaining a consistency result between an imaging center of the diagnostic beam and the isocenter position, and adjusting the fan beam CT assembly.

In an exemplary embodiment of the control method for a radiotherapy device, controlling the fan beam CT assembly to perform CT imaging on the phantom to be tested, and analyzing and obtaining the consistency result between the imaging center of the diagnostic beam and the isocenter position, further including: moving the center of the phantom to be tested to the isocenter position again; controlling the fan beam CT assembly to perform graphic guidance on the phantom to be tested, and through EPID image acquisition and analysis, obtaining a projection coordinate value of an image center position of the phantom to be tested in an EPID coordinate system; and by adjusting the fan beam CT assembly, correcting a deviation between the projection coordinate value of the image center position and a coordinate value of an EPID isocenter.

In an exemplary embodiment of the control method for a radiotherapy device, the radiotherapy device further includes a plurality of vibration sensors, and the control method further includes: in response to vibration data detected by one of the plurality of vibration sensors exceeding a first preset vibration threshold, controlling the rotation frame to enter the low-speed protection state.

In an exemplary embodiment of the control method for a radiotherapy device, the radiotherapy device includes at least four counterweight devices correspondingly arranged with the plurality of vibration sensors, each of the at least four counterweight devices includes a second driving device and a counterweight structure whose position is movable by the second driving device;

where the control method further includes: after entering the low-speed protection state due to the vibration data from the vibration sensor exceeding the first preset vibration threshold, adjusting, according to the vibration sensor that issues the vibration data, a counterweight position of the counterweight structure in the counterweight device corresponding to the vibration sensor.

In an exemplary embodiment of the control method for a radiotherapy device, the control method further includes: in response to the vibration data detected by the vibration sensor exceeding a second preset vibration threshold, controlling all power supplies of the radiotherapy device except a power supply of the rotation frame to be disconnected, controlling the rotation frame to gradually decelerate to enter the low-speed protection state, and powering off after a specific speed is reached.

In an exemplary embodiment of the control method for a radiotherapy device, the accelerator treatment head of the radiotherapy device includes a multi-leaf collimator, and the step of controlling the treatment beam assembly to return to the preset position further includes: returning the multi-leaf collimator so that movement directions of leaves of the multi-leaf collimator are parallel to the rotation axis of the rotation frame.

In an exemplary embodiment of the control method for a radiotherapy device, the multi-leaf collimator includes a pair of leaf carriages, and the pair of leaf carriages are movable relative to each other and carry the leaves. The step of controlling the treatment beam assembly to return to the preset position further includes: returning the pair of leaf carriages of the multi-leaf collimator so that the pair of leaf carriages return to mutually symmetrical positions.

In an exemplary embodiment of the control method for a radiotherapy device, the treatment beam assembly of the radiotherapy device includes jaws in pairs, where the step of controlling the treatment beam assembly to return to the preset position further includes: returning the jaws so that the jaws return to mutually symmetrical positions.

In an exemplary embodiment of the control method for a radiotherapy device, the radiotherapy device further includes a water cooling device for maintaining a constant temperature of the heat-generating components of the accelerator, and before switching from the treatment working state to the diagnostic working state, a water supply of the water cooling device is reduced to a preset supply value.

In an exemplary embodiment of the control method for a radiotherapy device, the radiotherapy device further includes a fan arranged on the rotation frame, and before switching from the treatment working state to the diagnostic working state, a rotational speed of the fan is reduced to a preset speed value.

As can be seen from the above, the at least one technical solution adopted in the embodiments of the present disclosure can achieve the following beneficial effects: on the one hand, the treatment beam assembly and the fan beam CT assembly can be driven simultaneously by the rotation frame, which can achieve both radiotherapy and CT imaging. The patient does not need to shift when switching between CT imaging and radiotherapy, so that centers of treatment and imaging intersect at one place, positioning is accurate. Traditional displacement compensation does not need to be considered during treatment, treatment can be directly implemented, which avoids image errors, and enables the treatment plan based on CT imaging to be more accurate. On the other hand, when switching from the treatment working state to the diagnostic working state, by controlling the treatment beam assembly to return to the preset position, components of the treatment beam assembly can be protected from damage caused by high-speed rotation, and the high-speed rotating device can be kept in a dynamic balance state, thereby protecting the overall device and enabling the equipment to reach a high rotational speed of up to 80 RPM for CT imaging. On the other hand, since the radiotherapy device integrates both the treatment beam assembly and the fan beam CT assembly, after the patient completes the CT diagnosis, the radiotherapy plan can be directly implemented without other preparation processes, thereby simplifying the implementation steps, and improving and optimizing the treatment process.

### BRIEF DESCRIPTION OF THE DRAWINGS

Various other advantages and benefits will become clear to those skilled in the art by reading the following detailed description of preferred embodiments. The accompanying drawings are merely for the purpose of illustrating the preferred embodiments and do not limit the present disclosure. Moreover, the same reference symbols are used to represent the same components throughout the drawings. In the attached drawings:
FIG. 1 is a perspective structural diagram illustrating an exemplary embodiment of a radiotherapy device.
FIG. 2 is a perspective structural diagram illustrating another exemplary embodiment of a radiotherapy device.
FIG. 3 is a schematic diagram illustrating an exemplary embodiment of a water cooling system of a radiotherapy device.
FIG. 4 illustrates specific control steps before switching from a treatment working state to a diagnostic working state.
FIG. 5 is a schematic structural diagram illustrating an exemplary embodiment of a multi-leaf collimator.
FIG. 6 illustrates steps of controlling a rotation frame to enter a low-speed protection state.
FIG. 7 is a schematic diagram illustrating arrangement of vibration sensors and counterweight devices.
FIG. 8 is a schematic structural diagram illustrating a fixed ring and an adjusting ring of a radiotherapy device.
FIG. 9 is a schematic structural diagram illustrating a first driving device of a radiotherapy device.

### Description of reference symbols:

10 fixed base
20 rotation frame
21 fixed plate
22 rotation axis
23 fixed ring
24 adjusting ring
25 set screw
26 installation arm
27 bolt assembly
271 vertical adjusting bolt
272 fixed bolt
273 washer
274 spring
30 treatment beam assembly
31 treatment beam
32 axis of treatment beam
33 accelerator treatment head
331 multi-leaf collimator
332 leaf
333 leaf carriage
35 treatment beam imaging unit
361 rotor
362 stator
40 fan beam CT assembly
41 diagnostic beam
42 axis of diagnostic beam
43 fan beam CT tube
44 shielding body
45 diagnostic beam detection unit
452 detector
50 pulse modulator
60 magnetron
72 water cooling device
722 water cooling tube
724 expansion tank
74 water distributor
76 inlet pipe
78 return pipe
80 vibration sensor
82 counterweight device

### DETAILED DESCRIPTION

In order to make the purpose, technical solutions, and advantages of the present disclosure more clear, the technical solutions of the present disclosure will be described in detail with reference to specific embodiments of the present disclosure and corresponding drawings. Obviously, the described embodiments are merely a part of the embodiments of the present disclosure, not all embodiments. Based on the embodiments in the present disclosure, all other embodiments obtained by ordinary technicians in the art without creative efforts belong to the protection scope of the present disclosure.

Herein, "schematic" means "serving as an instance, example or illustration", and any diagram or implementation described as "schematic" herein should not be interpreted as a more preferred or more advantageous technical solution.

In order to make the drawings simple and concise, only relevant parts of the present disclosure are schematically shown in each drawing, and they do not represent the actual structure of the product. In addition, in order to make the drawings simple, concise and easy to understand, in some drawings, only one of the components having the same structure or function is schematically drawn or marked.

The technical solutions provided by various embodiments of the present disclosure will be described in detail with reference to the accompanying drawings.

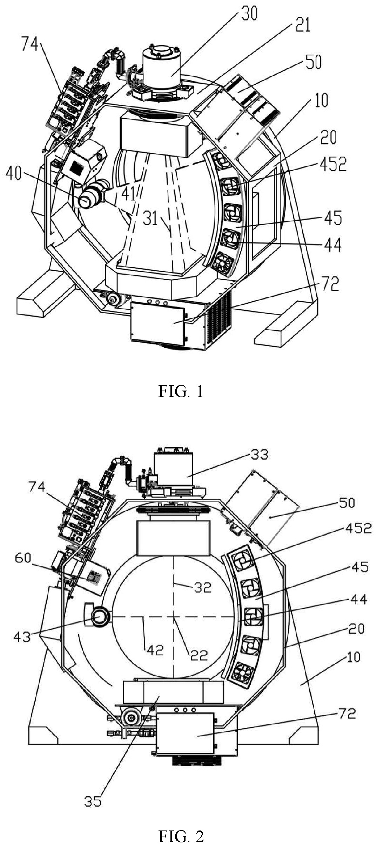

FIG. 1 is a perspective structural diagram illustrating an exemplary embodiment of a radiotherapy device. As shown in FIG. 1, the radiotherapy device includes a fixed base 10, a rotation frame 20, a treatment beam assembly 30, and a fan beam computed tomography (CT) assembly 40.

The rotation frame 20 is rotatably arranged on the fixed base 10, and the rotation frame 20 is provided with the treatment beam assembly 30 and the fan beam CT assembly 40, so that the rotation frame 20 can simultaneously drive the treatment beam assembly 30 and the fan beam CT assembly 40 to rotate together.

FIG. 1 shows a treatment beam 31 of the treatment beam assembly 30 with dashed lines, and a diagnostic beam 41 of the fan beam CT assembly 40 with dashed lines. After the treatment beam assembly 30 rotates with the rotation frame 20, an axis 32 of the treatment beam 31 (see FIG. 2) will rotate in a rotational plane. After the fan beam CT assembly 40 rotates with the rotation frame 20, an axis 42 of the diagnostic beam 41 (see FIG. 2) will also rotate in a rotational plane.

In the present disclosure, a rotation plane of the axis 32 of the treatment beam 31 is located and a rotation plane of the axis 42 of the diagnostic beam 41 is located coincide with each other, that is, they are located in the same plane, and the axis 32 of the treatment beam 31 and the axis 42 of the diagnostic beam 41 intersect at a rotation axis 22 of the rotation frame 20, as shown in FIG. 2. The axis in the present disclosure is defined as an imaginary line formed along a central emission direction of the treatment beam or the diagnostic beam.

After adopting the above design, the treatment beam assembly 30 and the fan beam CT assembly 40 can be driven simultaneously by the rotation frame 20, which can achieve both radiotherapy and CT imaging. Moreover, because the rotation plane where the axis of the treatment beam of the accelerator treatment head in the treatment beam assembly is located is coplanar with the rotation plane where the axis of the diagnostic beam of the fan beam CT assembly is located, and the axis 32 of the treatment beam 31 and the axis 42 of the diagnostic beam 41 intersect at the rotation axis 22 of the rotation frame 20, a patient does not need to shift when switching between CT imaging and radiotherapy, so that centers of treatment and imaging intersect at one place, positioning is accurate, and the treatment plan based on CT imaging is more accurate. Traditional displacement compensation does not need to be considered during treatment, treatment can be directly implemented, which can avoid image errors and achieve more accurate treatment. When switching from a treatment working state to a diagnostic working state, by controlling the treatment beam assembly to return to a preset position, components of the treatment beam assembly can be protected from damage caused by high-speed rotation, and the high-speed rotation can be kept in a dynamic balance state, thereby protecting the overall device and enabling the equipment to reach a high rotational speed of up to 80 RPM for CT imaging.

In addition, since the radiotherapy device integrates both the treatment beam assembly and the fan beam CT assembly, after the patient completes the CT diagnosis, the radiotherapy plan can be directly implemented without other preparation processes, thereby simplifying the implementation steps, and improving and optimizing the treatment process.

In the embodiments shown in FIG. 1 and FIG. 2, the treatment beam assembly 30 includes an accelerator treatment head 33, a pulse modulator 50, a magnetron 60, and a treatment beam imaging unit 35 arranged opposite to the accelerator treatment head 33. The treatment beam imaging unit 35, for example, employs a flat panel detector. The fan beam CT assembly 40 includes a fan beam CT tube 43 and a diagnostic beam detection unit 45 arranged opposite to the fan beam CT tube 43. The diagnostic beam detection unit 45, for example, as shown in the figures, employs a plurality of detectors 452 arranged in an arc shape. Moreover, the axis 32 of the treatment beam 31 is arranged perpendicular to the axis 42 of the diagnostic beam 41. Preferably, the treatment beam imaging unit 35 includes an electronic portal imaging device (EPID), which is an important device in accelerator quality control detection technology, and may further include a radiation detection device and a radiation signal processing device.

The above arrangement where the axis 32 of the treatment beam 31 and the axis 42 of the diagnostic beam 41 are perpendicular to each other can make the layout of the whole structure more reasonable, mainly considering the following two reasons.

Firstly, both the treatment beam assembly 30 and the fan beam CT assembly 40 are very heavy equipment. During the rotation of the rotation frame 20, especially during CT imaging which requires high-speed continuous rotation, it is necessary to properly arrange positional relationships between the accelerator treatment head 33, the treatment beam imaging unit 35, the fan beam CT tube 43, and the diagnostic beam detection unit 45, to ensure that a center of gravity is as close as possible to the rotation axis 22 of the rotation frame 20.

Secondly, the accelerator treatment head 33 usually operates at a megavolt (MV) level, and the fan beam CT tube 43 usually operates at a kilovolt (KV) level. When the accelerator treatment head 33 emits radiation or the fan beam CT tube 43 emits radiation, it may cause damage to other components. Therefore, the above arrangement can help avoid such damage as much as possible.

In the embodiment shown in FIG. 2, the pulse modulator 50 is arranged between the accelerator treatment head 33 and the diagnostic beam detection unit 45, and a pulse unit of the pulse modulator 50 is arranged along a radial direction pointing to the rotation axis 22. The magnetron 60 is arranged between accelerator treatment head 33 and the fan beam CT tube 43, and the magnetron 60 is arranged along a radial direction pointing to the rotation axis 22.

The pulse modulator 50 is used to control high-voltage pulses entering the accelerator treatment head 33. The pulse modulator 50 needs to be arranged on the rotation frame 20 together with the accelerator treatment head 33. The pulse modulator 50 is arranged along a radial direction pointing to the rotation axis 22, which is consistent with a main force direction after rotation, thereby protecting the internal structure of the pulse modulator 50.

The magnetron 60 is an electric vacuum device used to generate microwave energy. As an important component for radiotherapy, the magnetron 60 also needs to be installed on the rotation frame 20 together with the accelerator treatment head 33. The magnetron 60 is arranged along a radial direction pointing to the rotation axis 22, which can reduce deflection and movement of filaments inside the magnetron during high-speed rotation, thereby keeping the cathode position stable and improving the stability under high-voltage operation.

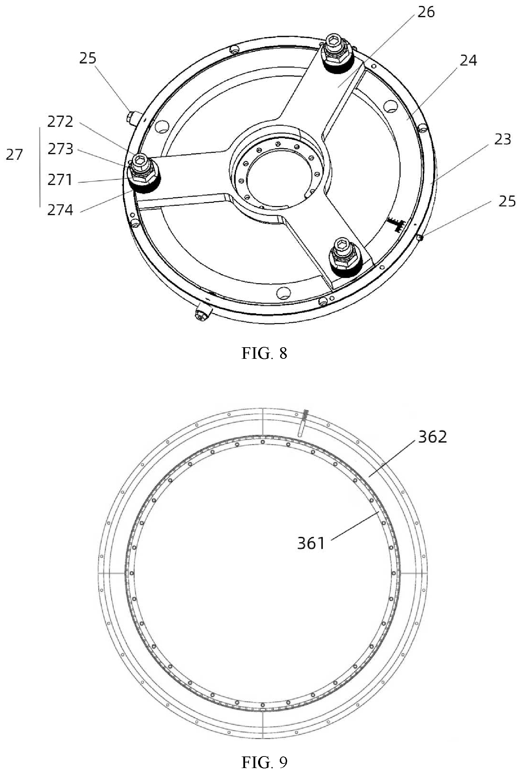

In the embodiments shown in FIG. 1 and FIG. 8, the accelerator treatment head 33 includes an acceleration tube, and the rotation frame 20 is provided with a fixed plate 21. The fixed plate 21 is provided with a through hole for the acceleration tube to pass through. The fixed plate 21 is provided with a fixed ring 23 protruding along an outer edge of the through hole, and an adjusting ring 24 embedded inside the fixed ring 23 and concentric with the fixed ring 23. The acceleration tube passes through the fixed ring 23 and the adjusting ring 24, and is connected to the adjusting ring 24 through an installation arm 26. The fixed ring 23 is provided with two pairs of set screws 25, and the two pairs of set screws 25 respectively pass through the fixed ring 23 and adjustably abut against an outer side wall of the adjusting ring 24, to achieve a position adjustment of the acceleration tube in a horizontal direction (X, Y direction).

As shown in FIG. 8, the installation arm 26 at least includes three cantilevers, a connecting ring formed jointly by ends of the three cantilevers is sleeved around an exterior of the acceleration tube, and the other ends of the three cantilevers are all arranged on the adjusting ring 24. Each cantilever is adjustably connected to the adjusting ring 24 through a bolt assembly 27, to achieve position adjustment of the acceleration tube in a vertical direction. The bolt assembly 27 at least includes a vertical adjusting bolt 271 and a fixed bolt 272 that passes through an interior of the vertical adjusting bolt 271 and is threadedly connected to the adjusting ring 24. Two ends of the vertical adjusting bolt 271 are respectively provided with washers 273, and a spring 274 is sleeved on an exterior of the vertical adjusting bolt 271.

It should be noted that in the present disclosure, a direction parallel to the ground and perpendicular to the rotation axis can be defined as the X direction, an extension direction of the rotation axis can be defined as the Y direction, and a direction perpendicular to the ground (orthogonal to the X direction and the Y direction) can be defined as a Z direction. Therefore, in this embodiment, there are arranged the two pairs of set screws on the fixed ring 23, which are distributed in the X direction and the Y direction respectively. A spring (such as set screws on the left side and front side in FIG. 8) is also installed in one of set screws holes of each pair of set screws in the X direction and the Y direction. The two pairs of set screws 25 respectively pass through the fixed ring 23 and abut against the outer side wall of the adjusting ring 24, and the springs can also abut against the outer side wall of the adjusting ring.

As shown in FIG. 8, during adjustment, the position of the acceleration tube in the horizontal direction can be adjusted by adjusting the four set screws in the horizontal direction. Due to interference from the frame, it is inconvenient to adjust the horizontal adjusting bolt on the left side. Therefore, the bolt on the right side can be directly adjusted, allowing the acceleration tube to be adjusted back by an elastic force of the spring. This enables position adjustment of the adjusting ring 24 and the acceleration tube connected to the adjusting ring 24 in the X direction and the Y direction, making adjustment more convenient and overcoming mechanical interference. Meanwhile, since the spring always keeps the horizontal adjusting bolt in a compressed state to press against the adjusting ring 24 during the adjustment, the adjustment accuracy of the horizontal adjusting bolt is improved.

Continuing to refer to FIG. 8, the three bolt assemblies 27 in the figure each include the vertical adjusting bolt 271 and the fixed bolt 272 that penetrates through the interior of the vertical adjusting bolt 271 and is threadedly connected to the adjusting ring 24. When the vertical adjusting bolt 271 is adjusted in place, the fixed bolt 272 is tightened to press against the vertical adjusting bolt 271 to secure the bolt assembly 27. An upper end and a lower end of the vertical adjusting bolt 271 are respectively provided with washers 273, that is, the lower end of the vertical adjusting bolt 271 abuts against the adjusting ring 24 through a pair of washers, and the fixed bolt 272 can also press against the upper end of the vertical adjusting bolt 271 through a pair of washers. Thus, by screwing the vertical adjusting bolt 271 in or out, heights of the three cantilevers in the Z direction can be adjusted, thereby adjusting a vertical height of the acceleration tube. Meanwhile, in this embodiment, mating surfaces of the washers are configured as curved surfaces, that is, the mating surface of the washer at the upper end of the vertical adjusting bolt is a curved surface, and the mating surface of the washer at the lower end of the vertical adjusting bolt against the adjusting ring 24 is also a curved surface. Therefore, during adjustment, the curved surfaces between the upper and lower pairs of washers can slide relative to each other, providing a fine-tuning function. In addition, the spring 274 sleeved outside the vertical adjusting bolt 271 provides a pressing effect to improve adjustment accuracy.

In addition, in the embodiment shown in FIG. 2, the radiotherapy device further includes a water cooling device 72, a water distributor 74, an inlet pipe 76, and a return pipe 78. The water cooling device 72 is arranged on the rotation frame 20 and arranged outside the treatment beam imaging unit 35 along a radial direction pointing to the rotation axis 22. The water distributor 74 is arranged on the rotation frame 20 and between the accelerator treatment head 33 and the fan beam CT tube 43, providing cooling water for heat-generating components of the accelerator. The inlet pipe 76 and the return pipe 78 are respectively connected between the water cooling device 72 and the water distributor 74, and the inlet pipe 76 and the return pipe 78 are arc-shaped pipes with their centers located on the rotation axis 22.

The water cooling device 72 can cool the accelerator treatment head 33, and placing the water cooling device 72 on the rotation frame 22 is more conducive to the arrangement of cooling pipelines. It should be understood that by adjusting the water temperature, in addition to cooling, it can also have a heating effect, thereby keeping equipment components always close to a suitable constant temperature.

In the above scheme, the water distributor 74 and the magnetron 60 are arranged between the accelerator treatment head 33 and the fan beam CT tube 43, the pulse modulator 50 is arranged between the accelerator treatment head 33 and the diagnostic beam detection unit 45, and the water cooling device 72 is also arranged on the rotation frame 22 and arranged outside the treatment beam imaging unit 35 along the radial direction pointing to the rotation axis 22. The above layout can make the overall weight distribution more balanced and ensure the dynamic balance of the entire equipment after rotation, that is, the arrangement of the water cooling device 72 itself can serve as a counterweight.

It should also be noted that the inlet pipe 76 and the return pipe 78 between the water cooling device 72 and the water distributor 74 are designed as arc-shaped pipes, and the centers of the arc-shaped pipes are located at the rotation axis 22 of the rotation frame 20. After adopting this design, during high-speed rotation, a water hammer phenomenon formed by water flow in the inlet pipe 76 and the return pipe 78 can be avoided.

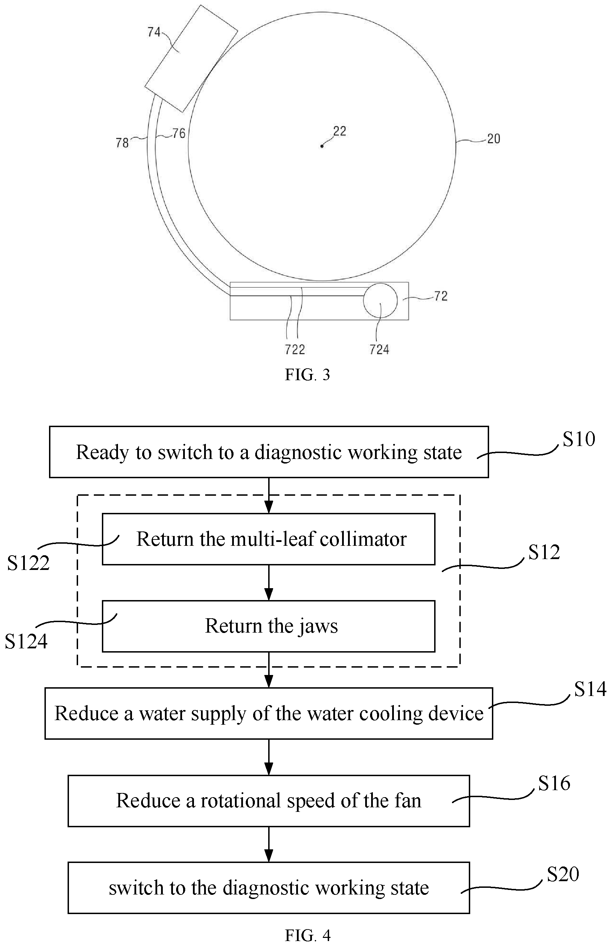

FIG. 3 is a schematic diagram illustrating an exemplary embodiment of a water cooling system of a radiotherapy device. As shown in FIG. 3, the water cooling device 72 includes water cooling tubes 722 arranged along the rotational tangential direction of the rotation frame 20. The water cooling device 72 also includes an expansion tank 724 connected to the water cooling tubes 722 and arranged parallel to the rotation axis 20. In existing water cooling devices, in order to reduce volume, the water cooling tubes are folded vertically, and the expansion tank 724 is also arranged vertically. The arrangement in the present disclosure also takes into account the direction of forces acting on the water cooling device 72 during high-speed rotation, ensuring greater stability of the entire device.

In addition to the above water cooling device for cooling the equipment components, the radiotherapy device is also equipped with an air cooling device. The air cooling device includes an outdoor unit, an indoor unit, and ducts of air conditioning equipment. The outdoor unit is of a variable frequency type. The indoor unit supplies cold air into an equipment casing through air ducts to cool the entire equipment. The return air is connected to the equipment casing and finally flows back to the indoor unit. The indoor unit can be installed on a ceiling of an equipment room or in a pit. A plurality of temperature sensors are arranged in the equipment casing. When one of the sensors detects that the temperature exceeds a certain threshold, a control system controls the air cooling device to increase a cold air supply volume and reduce a air supply temperature for rapid cooling. In normal operation, the air supply volume and air supply temperature of the air cooling device are different in the treatment working state and the diagnostic working state. In the diagnostic working state, the temperature is lower, the supply air temperature is lower, and the supply air volume is larger. When the radiotherapy device switches from the treatment working state to the diagnostic working state, the control system will control the air cooling device to adjust the temperature.

In the embodiments shown in FIG. 1 and FIG. 2, the radiotherapy device further includes a shielding body 44 that is movably arranged on a radial inner side of the diagnostic beam detection unit 45. The shielding body 44 has a shielding position capable of shielding the diagnostic beam detection unit 45 when the accelerator treatment head 33 is in operation, and an open position capable of exposing the diagnostic beam detection unit 44 when the fan beam CT tube 43 is in operation. For example, in some embodiments, the shielding body 44 has a stepped structure, with a thick end close to the treatment beam imaging unit 35, and then the thickness gradually decreases in a stepped manner. The shielding body 44 can be installed on a shielding body mounting seat. A driving motor is installed on a housing of the diagnostic beam detection unit 45, and the shielding body mounting seat is driven by the driving motor, which can drive the shielding body 44 to move back and forth in the Z direction along the rotation axis 22 of the rotation frame 20.

As described above, when the treatment beam assembly 30 and the fan beam CT assembly 40 are arranged on the same rotation frame 20, it is necessary to consider the problem of radiation damage to other devices, especially the accelerator treatment head 33 of the treatment beam assembly 30, which is usually at the megavolt (MV) level. During operation, the radiation will be reflected to other positions. Although the above problem has been avoided as much as possible by arranging the axis of the treatment beam perpendicular to the axis of the diagnostic beam, the protection of the diagnostic beam detection unit 45 can be further improved by arranging the shielding body 44. Moreover, the shielding body 44 is set in a stepped shape, which makes the protection of a strong reflection area close to the treatment beam imaging unit 35 stronger. For positions far away from the treatment beam imaging unit 35, the reflected radiation is smaller and does not require too thick, which can reduce the weight of the shielding body. This enables a reasonable layout of the treatment beam assembly 30, the fan beam CT assembly 40 and other components in a limited space, and can also enhance the protection of the fan beam CT assembly 40 during the operation of the treatment beam assembly 30. The thickness of the shielding body 44 can be designed with reference to "GBZ/T 201.2-2011 Radiation Shielding Specifications for Radiotherapy Room", and the present disclosure does not limit this.

In an example shown in this embodiment, the radiotherapy device further includes:
a first driving device configured to drive the rotation frame 20 to rotate;
where the first driving device includes a rotor 361 and a stator 362;
the rotor 361 is annular and drives the rotation frame 20 to rotate; the rotor 361 is provided with a plurality of magnets; the plurality of magnets are uniformly arranged along a circumferential direction of the rotor 361;
the stator 362 is annular, sleeved on outside the rotor 361, and fixedly connected to the fixed base 10; the stator 362 is provided with a plurality of sets of coils, and the plurality of sets of coils are uniformly arranged along a circumferential direction of the stator 362.

In addition, in the technical solution of the present disclosure, for the first driving device in the radiotherapy device, under the working state, the plurality of magnets on the rotor 361 can generate a corresponding magnetic field. When a certain current is introduced into the coils of the stator 362, a changing magnetic field will be generated, and this magnetic field will interact with the magnetic field generated by the rotor 361 to produce electromagnetic thrust, pushing the rotor 361 and the stator 362 to rotate. Since the stator 362 is fixed to the base, the rotor 361 will rotate under the action of electromagnetic thrust, thereby driving the rotation frame 20 fixed to the rotor 361 to also rotate, and causing the treatment beam assembly 30 and the fan beam CT assembly 40 to rotate around the central rotation axis. Therefore, when it is necessary to use the treatment beam assembly 30 or the fan beam CT assembly 40, the first driving device can be used to drive the rotation, driving the treatment beam assembly 30 or the fan beam CT assembly 40 to rotate to a corresponding position.

Since the first driving device can directly drive the rotation frame 20 without the need for gears, gearboxes, or other structures, the treatment beam assembly 30 and the fan beam CT assembly 40 can operate stably under variable speed, high speed, and ultra-low speed conditions, with a maximum rotational speed of up to 80 RPM.

The present disclosure also provides a control method for a radiotherapy device. The radiotherapy device at least includes a fixed base 10, a rotation frame 20, a treatment beam assembly 30, and a fan beam CT assembly 40 as shown in FIG. 1. The rotation frame 20 is rotatably arranged on the fixed base 10, and the rotation frame 20 is provided with the treatment beam assembly 30 and the fan beam CT assembly 40, so that the rotation frame 20 can simultaneously drive the treatment beam assembly 30 and the fan beam CT assembly 40 to rotate together. A rotation plane where an axis 32 of a treatment beam 31 from the treatment beam assembly 30 is located is coplanar with a rotation plane where an axis 42 of a diagnostic beam 41 from the fan beam CT assembly 40 is located, and the axis 32 of the treatment beam 31 and the axis 42 of the diagnostic beam 41 intersect at a rotation axis 22 of the rotation frame 20.

The treatment beam assembly 30 includes an accelerator treatment head 33 and a treatment beam imaging unit 35 arranged opposite to the accelerator treatment head 33. The treatment beam imaging unit 35, for example, employs a flat panel detector. The fan beam CT assembly 40 includes a fan beam CT tube 43 and a diagnostic beam detection unit 45 arranged opposite to the fan beam CT tube 43. The diagnostic beam detection unit 45, for example, as shown in the figures, employs a plurality of detectors 452 arranged in an arc shape. Moreover, the axis 32 of the treatment beam 31 is arranged perpendicular to the axis 42 of the diagnostic beam 41.

The radiotherapy device also includes a pulse modulator 50 and a magnetron 60. The pulse modulator 50 is arranged on the rotation frame 20 and between the accelerator treatment head 33 and the diagnostic beam detection unit 45. The pulse modulator 50 itself is arranged radially towards the rotation axis 22. The magnetron 60 is arranged on the rotation frame 20 and between the accelerator treatment head 33 and the fan beam CT tube 43. The magnetron 60 is arranged radially towards the rotation axis 22.

The control method can control the radiotherapy device to enter the treatment working state or the diagnostic working state. In the treatment working state, the rotation frame is controlled to drive the treatment beam assembly to move to a treatment position to achieve radiotherapy, and in the diagnostic working state, the rotation frame is controlled to drive the fan beam CT assembly to continuously rotate to achieve diagnostic imaging.

The control method further includes: before switching from the treatment working state to the diagnostic working state, the treatment beam assembly 30 is controlled to return to a preset position; and after switching to the diagnostic working state, it is detected whether the treatment beam assembly 30 is out of the preset position, and in response to detecting that the treatment beam assembly 30 is out of the preset position, the rotation frame 20 is controlled to slow down and enter a low-speed protection state.

As described earlier, the CT imaging process requires high-speed continuous rotation, and both the treatment beam assembly 30 and the fan beam CT assembly 40 are very heavy equipment. Therefore, after the treatment beam assembly 30 works, it is necessary to consider the impact of continuous high-speed rotation on the treatment beam assembly 30, as well as the impact of the treatment beam assembly 30 on the dynamic balance after high-speed rotation. The above control method takes into account these impacts, protecting the components of the treatment beam assembly from damage caused by high-speed rotation, while also maintaining a dynamic balance of the high-speed rotation, thereby protecting the overall device.

In addition, in the high-speed rotation process, if the treatment beam assembly moves away from its preset position, the rotation frame 20 will be controlled to slow down, causing the overall device to enter the low-speed protection state to prevent damage to the equipment due to the disruption of dynamic balance.

Referring to FIG. 4, which illustrates specific control steps before switching from the treatment working state to the diagnostic working state. FIG. 4 is merely a schematic illustration, and there is no specific order of steps in actual operating conditions. In addition, the treatment beam assembly 30 of the radiotherapy device will include jaws, the accelerator treatment head will include a multi-leaf collimator 331, and the radiotherapy device will include a water cooling device 72 and a fan located on the rotation frame 20.

As shown in FIG. 4, after discovering that the diagnostic working state is ready to be switched in step S10, the process can proceed to steps S12, S14, and S16, where the steps are not in any particular order. Finally, the process can proceed to step S20 to switch to the diagnostic working state. The steps S12, S14, and S16 are specifically as follows.

In step S12, the treatment beam assembly 30 is controlled to return to the preset position, which further includes steps S122 and S124.

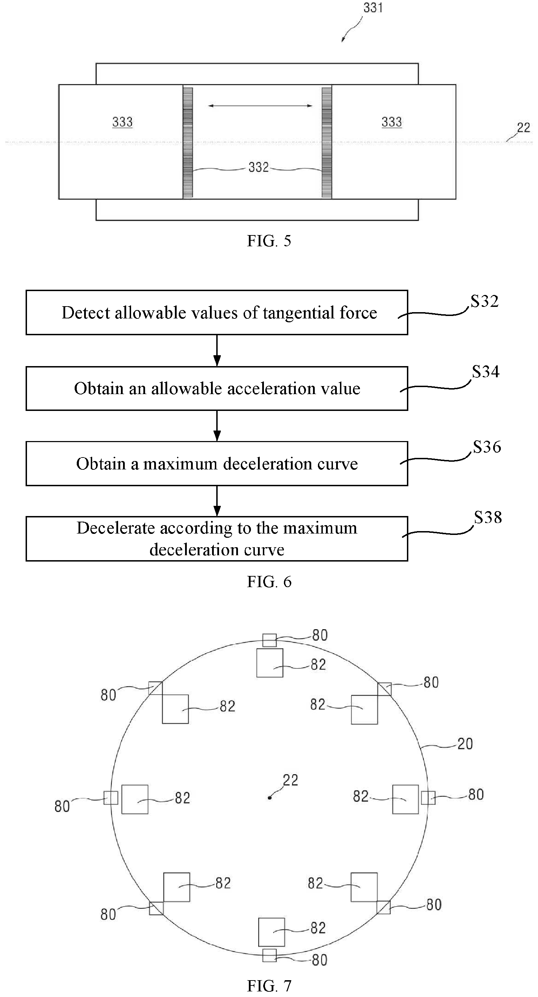

In step S122, the multi-leaf collimator 331 is returned. Referring to FIG. 1 and FIG. 5, the multi-leaf collimator 331 is located below the fixed plate 21, so that movement directions of leaves 332 of the multi-leaf collimator 331 (directions indicated by the double-headed arrow in the figure) are parallel to the rotation axis 22 of the rotation frame 20 (see FIG. 2). After returning, in the case of high-speed rotation, the movement directions of the leaves 332 are always perpendicular to a tangential direction of rotation, which not only protects the leaves 322 but also prevents displacement of the leaves 322. In addition, the step S122 may further include returning a pair of leaf carriages 333 of the multi-leaf collimator 331. When the leaf carriages 333 are in operation, they can move relative to each other and carry leaves 332. The return control causes the pair of leaf carriages 333 to drive the leaves 322 return to symmetrical positions, that is, to return them to symmetrical positions on both sides of a center line in a plane where the leaves 322 and the leaf carriages 333 are located, thereby reducing the impact on dynamic balance under high-speed rotation.

In step S124, the jaws of the treatment beam assembly 30 are returned. The jaws (not shown in the figure) are usually in pairs. The above step controls the paired jaws to return to mutually symmetrical positions to reduce the impact on dynamic balance under high-speed rotation.

In step S14, a water supply of the water cooling device 72 (refer to FIG. 2 and FIG. 3) is reduced to a preset supply value to reduce a water pressure and a flow rate of the water cooling system. After switching to the diagnostic working state, the treatment beam assembly 30 is not in operation, and the heat dissipation demand decreases. At this time, reducing the water supply of the water cooling device 72 can also ensure basic heat dissipation and reduce the impact of water flow on the treatment beam assembly 30 (mainly the accelerator treatment head) under high-speed rotation.

In step S16, a rotational speed of the fan (not shown in the figure) of the radiotherapy device is reduced to a preset speed value. The fan also plays a role in cooling the treatment beam assembly 30. After switching to the diagnostic working state, high-speed rotation will cause the fan to produce greater vibration and noise, which is prone to damage. Reducing rotational speed of the fan at this time can reduce the above vibration and noise, improve a service life of the fan, and enhance user experience.

After switching to the diagnostic working state, the control method of the present disclosure further includes the step of controlling the rotation frame 20 to slow down and enter the low-speed protection state. As shown in FIG. 6, this step further includes the following steps:
step S32, allowable force values of components arranged on the rotation frame in the rotational tangential direction are detected;
step S34, the allowable force values of the components are compared, and an allowable acceleration value of the rotation frame is obtained according to a minimum of the allowable force values;
step S36, a maximum deceleration curve of the rotation frame is obtained according to the allowable acceleration value; and
step S38, the rotation frame is controlled to reduce its rotational speed according to the maximum deceleration curve and enter the low-speed protection state.

The above control method can avoid the impact of sudden deceleration on the components on the rotation frame 20 during high-speed rotation, and this deceleration process can also form a buffer for an emergency stop of the subsequent rotation action.

Besides the case where the treatment beam assembly is detected to have moved away from its preset position and the rotation frame can be controlled to enter the low-speed protection state, there are other situations in which the rotation frame can also be controlled to enter the low-speed protection state. For example, as shown in FIG. 7, the radiotherapy device can also be provided with a plurality of vibration sensors 80, and the vibration sensors 80 can be evenly arranged around the rotation axis 22 of the rotation frame 20. Each vibration sensor 80 can sense vibration data at the corresponding position. Once vibration data of any vibration sensor 80 exceeds a first preset vibration threshold, the rotation frame 20 is controlled to enter the low-speed protection state, so as to protect the radiotherapy device.

It can be understood that before entering the imaging state, the rotor in the radiotherapy device will enter a stationary state. After the shielding body 44 returns to the open position and the leaf carriages 333 return to the symmetrical positions so that the leaves 322 move to the directions parallel to the rotation axis, the speed is gradually increased and enters a high-speed state to reach a required position. Therefore, the radiotherapy device can achieve static balance through calibration operations in a low-speed or stationary state. However, in a high-speed state, due to the large weight of the accelerator treatment head 33, the treatment beam imaging unit 35, the fan beam CT tube 43, and the diagnostic beam detection unit 45, these components will expand and deviate under centrifugal force. Since the weights, structures, and strengths of the components are different, their deformations are different, resulting in large vibration of the entire machine at high speed. To solve the above problem, in some embodiments, a plurality of counterweight devices 82 corresponding to the respective vibration sensors 80 can be provided to adjust the counterweight and realize dynamic balance adjustment of the device.

Referring to FIG. 7, the control method further includes, after entering the low-speed protection state due to the vibration data from the vibration sensor exceeding the first preset vibration threshold, according to the vibration sensor that issues the vibration data, a counterweight position of a counterweight structure in the counterweight device 82 in the corresponding position is adjusted, so as to dynamically adjust the overall counterweight on the rotation frame 20 to meet requirements of high-speed rotation.

The control method further includes: in response to the vibration data detected by the vibration sensor exceeding a second preset vibration threshold, all power supplies of the radiotherapy device except a power supply of the rotation frame are controlled to be disconnected, the rotation frame is controlled to gradually decelerate to enter the low-speed protection state, and the power is cut off after a specific speed is reached. Therefore, in emergency situations, the rotation frame can be slowed down to a low-speed state and then shut down, protecting the radiotherapy device while enhancing the safety of use.

In some preferred embodiments, four sets of counterweight devices 82 are arranged on the rotation frame. Each of the four sets of counterweight devices 82 includes a second driving device and a counterweight structure whose position is movable by the second driving device. The four sets of counterweight devices 82 are respectively installed at appropriate positions close to the accelerator treatment head 33, the treatment beam imaging unit 35, the fan beam CT tube 43, and the diagnostic beam detection unit 45.

For example, the second driving device is provided with two parallel first worms arranged radially along the rotation frame 20. Each first worm is provided with a first sliding block, and a second worm arranged perpendicular to the first worm is arranged between the two first sliding blocks. The second worm is provided with a second sliding block. The second sliding block is hollow and internally provided with a plurality of threaded holes (for example, four threaded holes). Each threaded hole is screwed with a third sliding block. These third sliding blocks are lifting blocks evenly arranged along an axial direction of the rotation frame 20. Each third sliding block is driven by a rotating motor to realize self-rotation, thereby achieving self-lifting along the threaded hole. In addition, each rotating motor is provided with a brake to prevent movement during high-speed rotation.

The advantages of arranging the counterweight devices in the embodiments of the present disclosure mainly lie in: on the one hand, by configuring different numbers and positions of lifting blocks in each second sliding block, the weight can be adjusted more flexibly, which is more conducive to balancing of the components with different weights such as the treatment head, thus enabling static balance adjustment of the device in a static state. On the other hand, the speed can be reduced before the state switching, and the positions of the counterweight structures in the four counterweight devices can be adjusted based on real-time detection results of the vibration sensors, so as to finally reduce the vibration to within the allowable error range. In the adjustment process, the position adjustment of the first sliding block and the second sliding block in the radial/perpendicular-to-radial direction directions can be achieved by driving the worm through the rotating motor, and the position adjustment of the third sliding block in the axial direction can be achieved by driving the third sliding block to self-rotate and lift through the rotating motor, so as to accurately adjust a center of gravity of the lifting block in the axial direction, thereby more accurately achieving the dynamic balance adjustment of the device.

The control method also includes: before entering the treatment working state or the diagnostic working state, online monitoring is performed to determine that the axis of the treatment beam and the axis of the diagnostic beam intersect at the rotation axis of the rotation frame. In addition to the above situations, online monitoring can also be conducted at regular intervals, times, or as needed. There are no restrictions here.

It can be understood that since the radiotherapy device in the present disclosure is calibrated before leaving the factory, the axis of the treatment beam, the axis of the diagnostic beam, and the rotation axis ultimately intersect at a center of rotation. That is, an isocenter point of the treatment beam and a center of the diagnostic beam on an isocenter plane are made to fall at an isocenter position. After the adjustment is completed, the isocenter position can be pre-marked. Each time a patient undergoes treatment or diagnostic imaging, a target area position will be moved to the pre-marked isocenter position.

Therefore, the purpose of setting up online monitoring function in the present disclosure is to determine whether the treatment beam and the diagnostic beam intersect at the rotation axis before treatment and imaging, that is, whether the center of the treatment beam and the center of the imaging beam are located at the isocenter, and to calibrate deviations existing in the respective components of the radiotherapy device by integrating various measurement indicators. For example, first it is measured whether the treatment bed has a positioning error. If not, a Winston-Lutz test (the Winston-Lutz test is used for measuring and verifying the isocenter point) is performed to analyze and obtain the consistency between the center of the treatment beam and the isocenter. Then, the consistency between the center of the imaging beam and the isocenter is analyzed through imaging. Therefore, through the examinations before diagnosis and treatment, it is ensured that the center of the imaging beam and the center of the treatment beam intersect at the isocenter point, thus ensuring the accuracy of treatment and imaging and fulfilling an original design intention of the equipment.

The specific measurement process includes but is not limited to the following situations: a numerical deviation of a physical distance (a distance between an imaging center of the treatment beam imaging unit and the isocenter position) measured by the treatment beam imaging unit is measured, and it is determined whether there is an imaging error in the treatment beam imaging unit; it is measured whether there is a positioning accuracy deviation; the consistency between a radiation center of the treatment beam and the isocenter position (that is, an isocenter position of the treatment beam radiation) is obtained through the analysis of the treatment test, and the consistency between the imaging center of the diagnostic beam and the isocenter position is analyzed through CT imaging testing.

After the above various measurements are completed, the numerical deviation of physical distance, the positioning accuracy deviation, and consistency results can be comprehensively used to register various components such as the treatment beam assembly and the fan beam CT assembly. For example, if the center of the treatment beam deviates from the rotation axis, the acceleration tube can be adjusted through an acceleration tube adjustment device (such as adjusting positions of the adjusting ring and cantilevers) to ensure that the axis of the treatment beam intersects with the isocenter position (the isocenter position), and ensure that the overall device is in a static balance state.

In addition, the present disclosure can also measure and adjust other components in the device, such as measuring accuracy of the movement speed of the multi-leaf collimator, a positioning accuracy deviation of the leaves, and registering based on deviation results, which will not be elaborated here. The above content is only an exemplary description and cannot be understood as a limitation on the present disclosure.

In some implementations, online monitoring includes: a phantom to be tested is installed at a fixed position on a treatment bed and a center of the phantom to be tested is moved to a pre-calibrated isocenter position; the treatment beam assembly is controlled to image the phantom to be tested, and a numerical deviation of a physical distance between the imaging center of the treatment beam imaging unit and the pre-calibrated isocenter position is obtained by analysis.

In some implementations, online monitoring also includes: a phantom to be tested is installed at a fixed position on a treatment bed and a center of the phantom to be tested is moved to a pre-calibrated isocenter position; the treatment bed is controlled to perform multiple shifts along different directions, and imaging is performed through the treatment beam assembly after each movement.

In the specific operation, the center of the phantom to be tested can be moved to a laser virtual center (located outside the machine) first. After other initial operations, the treatment bed automatically repositions to moves the center of the phantom to be tested from the laser virtual center to the isocenter position. Then, the treatment bed is controlled to perform multiple shifts along different directions, and imaging is performed through the treatment beam assembly after each movement.

Through superposition results of the current imaging with the previous imaging, positioning accuracy deviations of the treatment bed in different directions are obtained by analysis respectively; the position of the treatment bed is adjusted according to numerical deviations and positioning accuracy deviations, and the center of the phantom to be tested is moved to the pre-calibrated isocenter position again; the treatment beam assembly is controlled to perform Winston-Lutz test on the phantom to be tested, a consistency result between the radiation center of the treatment beam and the isocenter position is analyzed and obtained, and the treatment beam assembly is registered.

It can be understood that multiple shifts of the treatment bed need to be carried out in opposite directions, and movement distances of the treatment bed in opposite directions should be kept consistent, so that the multiple movement positions are symmetrical to each other. For example, steps for determining the positioning accuracy deviation in this embodiment specifically are as follows.

The treatment bed is moved in the Y direction by a first distance (for example, a distance of m), and a first imaging is performed on the phantom to be tested through the treatment beam assembly. Through the first imaging, a positioning accuracy deviation of the treatment bed moving in the Y direction by the first distance is analyzed. The treatment bed is moved in an opposite direction to the Y direction by a distance of 2m (that is, return to the initial position by a distance of m and then move in the opposite direction by a distance of m), and a second imaging is performed on the phantom to be tested through the treatment beam assembly. The first imaging and the second imaging are superimposed, and a positioning accuracy deviation of the treatment bed moving in the opposite direction to the Y direction by the distance of 2m is analyzed.

The treatment bed is moved in the X direction by a second distance (for example, a distance of n), and a third imaging is performed on the phantom to be tested through the treatment beam assembly. By superimposing the third imaging with the second imaging, a positioning accuracy deviation of the treatment bed moving in the X direction by the second distance is analyzed. The treatment bed is moved in an opposite direction to the X direction by a distance of 2n, a fourth imaging is performed on the phantom to be tested through the treatment beam assembly. By superimposing the fourth imaging with the third imaging, and a positioning accuracy deviation of the treatment bed moving in the opposite direction to the X direction by the distance of 2n is analyzed.

The treatment bed is moved in the Z direction by a third distance (for example, a distance of L), and a fifth imaging is performed on the phantom to be tested through the treatment beam assembly. By superimposing the fifth imaging with the fourth imaging, a positioning accuracy deviation of the treatment bed moving in the Z direction by the third distance is analyzed. The treatment bed is moved in an opposite direction to the Z direction by a distance of 2L, a sixth imaging is performed on the phantom to be tested through the treatment beam assembly. By superimposing the sixth imaging with the fifth imaging, and a positioning accuracy deviation of the treatment bed moving in the opposite direction to the Z direction by the distance of 2L is analyzed.

Further, the treatment beam assembly is controlled to acquire an electronic portal imaging device (EPID) image of the phantom to be tested, the center of the phantom to be tested is registered to an image center position in the EPID image, and a registration result of the phantom to be tested is obtained; the treatment beam assembly is controlled to perform a Winston-Lutz test on the phantom to be tested, and in combination with the registration result, a consistency result between the radiation center of the treatment beam and the isocenter position is analyzed and obtained, so as to adjust the treatment beam assembly.

When the radiation center of the treatment beam deviates from the isocenter position, the acceleration tube adjustment device is used to adjust the acceleration tube so that the beam emitted from the treatment beam is located at the isocenter. When center coordinates of the imaged phantom (that is, projection coordinates of an image center position of the phantom to be tested in an EPID coordinate system) are consistent with projection coordinates of the isocenter on the EPID, it is considered that the phantom to be tested has moved to the isocenter.

CT imaging is performed on the phantom to be tested through the fan beam CT assembly, and the consistency of the imaging center of the diagnostic beam and the isocenter position is analyzed. This further includes: the center of the phantom to be tested is moved to the isocenter position again; the fan beam CT assembly is controlled to emit a beam for graphic guidance on the phantom to be tested, and through EPID image acquisition and analysis, a projection coordinate value of the image center position of the phantom to be tested in the EPID coordinate system is obtained; and by adjusting the fan beam CT assembly, a deviation between the projection coordinate value of the image center position and a coordinate value of an EPID isocenter (that is, a projection coordinate value of the isocenter on the EPID) is corrected.

In specific implementation, the isocenter position is measured by a specific fixture and calibrated through a metal marking point. Therefore, projection coordinates of the marking point of the isocenter position in the EPID image can be obtained by through emitting of the treatment beam. For example, image analysis software is used to obtain coordinates (a, b) of the metal marking point on the EPID image. By moving the phantom to be tested and acquiring the EPID image, a metal ball at the center of the phantom to be tested reaches the coordinates (a, b), and at this point, it is considered that the center of the phantom to be tested is at the isocenter position. Afterwards, a kilovolt image is acquired through exposure of the fan beam CT assembly, the center of the phantom to be tested is registered to the image center in the EPID image under the graphic guidance of the CT assembly, and the registration result, that is, a deviation between the image center position and the isocenter position, is observed in real time. If the deviation value is within a preset error range, no adjustment is needed; if the deviation value does not meet the preset error range, the fan beam CT tube needs to be adjusted, and the above steps are repeated until the deviation requirements are met.

Therefore, through the online monitoring before diagnosis and treatment, it can be ensured that the axis of the diagnostic beam and the axis of the treatment beam intersect at the isocenter position, thereby ensuring the accuracy of treatment and imaging, avoiding image errors, and making treatment more accurate.

It should be noted that each step of the control method for the radiotherapy device mentioned above can be used to control the radiotherapy device provided in the previous embodiments. The relevant explanations about the radiotherapy device are all applicable to the control method for the radiotherapy device, and will not be repeated here.

The above is merely specific implementations of the present disclosure. Under the above teachings of the present disclosure, those skilled in the art can make other improvements or modifications based on the above embodiments. Those skilled in the art should understand that the above specific description is only for better explaining the purpose of the present disclosure, and the protection scope of the present disclosure should be based on the protection scope of the claims.

## Claims

1. A radiotherapy device, comprising:
a fixed base;
a rotation frame rotatably arranged on the fixed base;
a treatment beam assembly arranged on the rotation frame, wherein the treatment beam assembly comprises an accelerator treatment head, a pulse modulator, a magnetron, and a treatment beam imaging unit arranged opposite to the accelerator treatment head;
a fan beam computed tomography (CT) assembly arranged on the rotation frame, wherein the fan beam CT assembly comprises a fan beam CT tube and a diagnostic beam detection unit arranged opposite to the fan beam CT tube;
wherein a rotation plane where an axis of a treatment beam of the accelerator treatment head is located is coplanar with a rotation plane where an axis of a diagnostic beam of the fan beam CT assembly is located, the axis of the treatment beam and the axis of the diagnostic beam intersect at a rotation axis of the rotation frame, and the axis of the treatment beam is perpendicular to and coplanar with the axis of the diagnostic beam;
the pulse modulator is arranged between the accelerator treatment head and the diagnostic beam detection unit, and a pulse unit of the pulse modulator is arranged along a radial direction pointing to the rotation axis;
the magnetron is arranged between the accelerator treatment head and the fan beam CT tube, and is arranged along a radial direction pointing to the rotation axis; and
an online monitoring module configured to determine that the axis of the treatment beam and the axis of the diagnostic beam intersect at the rotation axis of the rotation frame before entering a treatment working state or a diagnostic working state.

2. The radiotherapy device according to claim 1, further comprising:
a water cooling device arranged on the rotation frame and arranged outside the treatment beam imaging unit along a radial direction pointing to the rotation axis;
a water distributor arranged on the rotation frame and between the accelerator treatment head and the fan beam CT tube, providing cooling water for heat-generating components of an accelerator; and
an inlet pipe and a return pipe connected between the water cooling device and the water distributor, wherein the inlet pipe and the return pipe are arc-shaped pipes centered on the rotation axis.

3. The radiotherapy device according to claim 2, wherein the water cooling device comprises:
water cooling tubes arranged along a rotational tangential direction of the rotation frame, and
an expansion tank arranged parallel to the rotation axis and connected to the water cooling tubes.

4. The radiotherapy device according to claim 1, further comprising a shielding body movably arranged on a radial inner side of the diagnostic beam detection unit, wherein the shielding body has a shielding position capable of shielding the diagnostic beam detection unit when the accelerator treatment head is in operation, and an open position capable of exposing the diagnostic beam detection unit when the fan beam CT tube is in operation;
a thickness of the shielding body decreases in a stepped manner from one end close to the treatment beam imaging unit.

5. The radiotherapy device according to claim 1, wherein the accelerator treatment head comprises an acceleration tube;
the rotation frame is provided with a fixed plate, and the fixed plate is provided with a through hole for the acceleration tube to pass through;
the fixed plate is provided with a fixed ring protruding along an outer edge of the through hole, and an adjusting ring embedded inside the fixed ring and concentric with the fixed ring; the acceleration tube passes through the fixed ring and the adjusting ring, and is connected to the adjusting ring through an installation arm;
the fixed ring is provided with two pairs of set screws, wherein the two pairs of set screws respectively pass through the fixed ring and adjustably abut against an outer side wall of the adjusting ring, to adjust a position of the acceleration tube in a horizontal direction;
the installation arm at least comprises three cantilevers, a connecting ring formed jointly by ends of the three cantilevers is sleeved around an exterior of the acceleration tube, and the other ends of the three cantilevers are all arranged on the adjusting ring;
each of the three cantilevers is adjustably connected to the adjusting ring through a bolt assembly, to adjust a position of the acceleration tube in a vertical direction; the bolt assembly at least comprises a vertical adjusting bolt and a fixed bolt that passes through an interior of the vertical adjusting bolt and is threadedly connected to the adjusting ring; two ends of the vertical adjusting bolt are respectively provided with washers, and a spring is sleeved on an exterior of the vertical adjusting bolt.

6. The radiotherapy device according to claim 1, further comprising:
a first driving device configured to drive the rotation frame to rotate;
wherein the first driving device comprises a rotor and a stator;
the rotor is annular and drives the rotation frame to rotate; the rotor is provided with a plurality of magnets; the plurality of magnets are uniformly arranged along a circumferential direction of the rotor;
the stator is annular, sleeved on outside the rotor, and fixedly connected to the fixed base; the stator is provided with a plurality of sets of coils, and the plurality of sets of coils are uniformly arranged along a circumferential direction of the stator.

7. A control method for a radiotherapy device, used for controlling the radiotherapy device according to any one of claims 1 to 6, the control method comprising:
controlling the radiotherapy device to enter the treatment working state, and in the treatment working state, controlling the rotation frame to drive the treatment beam assembly to move to a treatment position for radiotherapy;
controlling the radiotherapy device to enter the diagnostic working state, and in the diagnostic working state, controlling the rotation frame to drive the fan beam CT assembly to continuously rotate for diagnostic imaging;
wherein the control method further comprises:
before switching from the treatment working state to the diagnostic working state, controlling the treatment beam assembly to return to a preset position; and
after switching to the diagnostic working state, detecting whether the treatment beam assembly is out of the preset position, and in response to detecting that the treatment beam assembly is out of the preset position, controlling the rotation frame to slow down and enter a low-speed protection state;
the control method further comprises:
before entering the treatment working state or the diagnostic working state, performing online monitoring to determine that the axis of the treatment beam and the axis of the diagnostic beam intersect at the rotation axis of the rotation frame.

8. The control method according to claim 7, wherein the step of controlling the rotation frame to enter the low-speed protection state further comprises:
detecting allowable force values of components arranged on the rotation frame in the rotational tangential direction;
obtaining an allowable acceleration value of the rotation frame according to a minimum of the allowable force values;
obtaining a maximum deceleration curve of the rotation frame according to the allowable acceleration value; and
controlling the rotation frame to reduce its rotational speed according to the maximum deceleration curve and enter the low-speed protection state.

9. The control method according to claim 7, wherein the online monitoring comprises:
installing a phantom to be tested at a fixed position on a treatment bed and moving a center of the phantom to be tested to a pre-calibrated isocenter position;
controlling the treatment bed to perform multiple shifts along different directions, and performing imaging through the treatment beam assembly after each movement;
controlling the treatment beam assembly to acquire an electronic portal imaging device (EPID) image of the phantom to be tested, registering the center of the phantom to be tested to an image center position in the EPID image, and obtaining a registration result of the phantom to be tested;
controlling the treatment beam assembly to perform a Winston-Lutz test on the phantom to be tested, and in combination with the registration result, analyzing and obtaining a consistency result between a radiation center of the treatment beam and the isocenter position, so as to adjust the treatment beam assembly; and
controlling the fan beam CT assembly to perform CT imaging on the phantom to be tested, and analyzing and obtaining a consistency result between an imaging center of the diagnostic beam and the isocenter position, so as to adjust the fan beam CT assembly.

10. The control method according to claim 9, wherein controlling the fan beam CT assembly to perform CT imaging on the phantom to be tested, and analyzing and obtaining the consistency result between the imaging center of the diagnostic beam and the isocenter position, further comprising:
moving the center of the phantom to be tested to the isocenter position again;
controlling the fan beam CT assembly to emit a beam for graphic guidance on the phantom to be tested, and through EPID image acquisition and analysis, obtaining a projection coordinate value of an image center position of the phantom to be tested in an EPID coordinate system; and
by adjusting the fan beam CT assembly, correcting a deviation between the projection coordinate value of the image center position and a coordinate value of an EPID isocenter.

11. The control method according to claim 7, wherein the radiotherapy device further comprises a plurality of vibration sensors, and the control method further comprises: in response to vibration data detected by one of the plurality of vibration sensors exceeding a first preset vibration threshold, controlling the rotation frame to enter the low-speed protection state.

12. The control method according to claim 11, wherein the radiotherapy device further comprises at least four counterweight devices correspondingly arranged with the plurality of vibration sensors, each of the at least four counterweight devices comprises a second driving device and a counterweight structure whose position is movable by the second driving device;
wherein the control method further comprises:
after entering the low-speed protection state due to the vibration data from the vibration sensor exceeding the first preset vibration threshold, adjusting, according to the vibration sensor that issues the vibration data, a counterweight position of the counterweight structure in the counterweight device corresponding to the vibration sensor.

13. The control method according to claim 11, further comprising:
in response to the vibration data detected by the vibration sensor exceeding a second preset vibration threshold, controlling all power supplies of the radiotherapy device except a power supply of the rotation frame to be disconnected, controlling the rotation frame to gradually decelerate to enter the low-speed protection state, and powering off after a specific speed is reached.

14. The control method according to claim 7, wherein the accelerator treatment head of the radiotherapy device comprises a multi-leaf collimator, wherein the step of controlling the treatment beam assembly to return to the preset position further comprises:
returning the multi-leaf collimator so that movement directions of leaves of the multi-leaf collimator are parallel to the rotation axis of the rotation frame.

15. The control method according to claim 14, wherein the multi-leaf collimator comprises a pair of leaf carriages, and the pair of leaf carriages are movable relative to each other and carry the leaves, wherein the step of controlling the treatment beam assembly to return to the preset position further comprises:
returning the pair of leaf carriages of the multi-leaf collimator so that the pair of leaf carriages return to mutually symmetrical positions.

16. The control method according to claim 7, wherein the treatment beam assembly of the radiotherapy device comprises jaws in pairs, wherein the step of controlling the treatment beam assembly to return to the preset position further comprises:
returning the jaws so that the jaws return to mutually symmetrical positions.

17. The control method according to claim 7, wherein the radiotherapy device further comprises a water cooling device for maintaining a constant temperature of the heat-generating components of the accelerator, wherein before switching from the treatment working state to the diagnostic working state, the method further comprises:
reducing a water supply of the water cooling device to a preset supply value.

18. The control method according to claim 7, wherein the radiotherapy device further comprises a fan arranged on the rotation frame, wherein before switching from the treatment working state to the diagnostic working state, the method further comprises:
reducing a rotational speed of the fan to a preset speed value.
